# FASCICULE DE BREVET EUROPEEN

(11) **EP 1 295 253 B1**
(45) Date de publication et mention de la délivrance du brevet: **07.01.2009**
(21) Numéro de dépôt: 01949531.6
(22) Date de dépôt: 26.06.2001
(51) Int. Cl.: G06T 1/00

(54) **PROCEDE ET DISPOSITIF DE MAQUILLAGE OU DE COLORATION**
VORRICHTUNG UND VERFAHREN ZUM SCHMINKEN ODER FÄRBEN
METHOD AND DEVICE FOR MAKE-UP OR COLOURING

(30) Priorité: 26.06.2000 FR 0008175
(43) Date de publication de la demande: 26.03.2003
(73) Titulaire: L'ORÉAL, 75008 Paris (FR)
(72) Inventeur: DAUGA, Christophe, F-92300 Levallois-Perret (FR); QUINN, Francis, Xavier, F-75005 Paris (FR)
(74) Mandataire: Casalonga, Axel
(86) Numéro de dépôt international: PCT/FR2001/002021
(87) Numéro de publication internationale: WO 2002/001499

(56) Documents cités:
- FR-A- 2 728 982
- US-A- 4 160 271
- US-A- 5 836 872

## Description

La présente invention concerne le maquillage, la coloration ou la réalisation de dessins sur une partie du corps humain.

La technique du tatouage est très anciennement connue et consiste à insérer sous l'épiderme des substances colorantes, de façon indélébile.

Le tatouage permet d'obtenir un dessin en couleur de très haute qualité, mais nécessite une intervention délicate, notamment en terme de risque sanitaire en raison de l'incision de la peau. Il en résulte que de nombreuses personnes renoncent à se faire tatouer en raison de l'indélébilité et de l'aspect chirurgical de l'opération.

On connaît également des produits de maquillage qui offrent de façon temporaire un certain effet couvrant et peuvent modifier substantiellement l'aspect de la partie du corps qu'ils recouvrent. Le document US 5 785 960 décrit un appareil pour fabriquer une composition de coloration de la peau à partir d'une lecture de la coloration de la peau.

Les produits de maquillage sont d'application aisée, mais leur tenue ne dépasse pas quelques heures, surtout par temps chaud et/ou humide.

Par ailleurs, les produits de maquillage cherchent à imiter l'aspect d'une peau normale et n'offrent pas de liberté de dessin.

Récemment, sont apparus des petits tampons permettant d'appliquer un dessin sur la peau par décalcomanie. La durabilité du dessin sur la peau est de l'ordre de quelques jours. Mais, aucune liberté de dessin n'est offerte. L'utilisateur n'effectue qu'une reproduction du dessin présent sur le tampon.

La présente invention est définie par les revendications et propose de remédier aux inconvénients évoqués ci-dessus.

La présente invention propose un procédé de réalisation de dessins, de maquillage ou de coloration temporaire, sans incision de la peau, capable de tenir compte des caractéristiques locales de la peau et offrant une totale liberté de dessin.

La présente invention propose aussi un dispositif de mise en oeuvre du procédé ci dessus. Le dispositif comprend un moyen de positionnement de ladite partie, un moyen de prise d'images, un moyen d'analyse d'images pour obtenir les caractéristiques locales de ladite partie, et un moyen d'application de produits de traitement sur ladite partie en fonction desdites caractéristiques locales.

Le procédé automatisé de réalisation de dessins, de maquillage ou de coloration, selon un aspect de l'invention, comprend les étapes définies dans la revendication 1.

Ainsi, l'invention offre les avantages de liberté de dessin du tatouage traditionnel, la facilité d'utilisation du maquillage et une durabilité moyenne qui peut être facilement contrôlée selon les désirs de l'utilisateur, de quelques heures à une vingtaine de jours. Le fait de tenir compte des caractéristiques locales de ladite partie augmente encore la qualité visuelle du dessin obtenu, notamment pour jouer sur des effets tridimensionnels ou de perspective ou encore optiques, permettant de rendre invisibles certaines imperfections cutanées.

On entend ici par partie du corps , notamment humain, la peau, par exemple du visage, d'un membre..., le cuir chevelu, les muqueuses, les semi-muqueuses, les fibres kératiniques, par exemple les cils, les sourcils, les cheveux, les ongles et les poils.

On entend ici par image une représentation bidimensionnelle de l'apparence d'un objet composé d'éléments unitaires à analyser, la zone d'analyse peut se limiter à un seul élément (par exemple un pixel).

La présente invention sera mieux comprise à l'étude de la description détaillée de quelques modes de réalisation pris à titre d'exemples nullement limitatifs et illustrés par les dessins annexés, sur lesquels :
- la figure 1 est une vue schématique d'un dispositif, selon un mode de réalisation de l'invention;
- la figure 2 est une vue de détail de la figure 1;
- la figure 3 est une vue schématique selon un autre mode de réalisation de l'invention;
   et la figure 4 est un diagramme montrant les étapes d'acquisition d'image dans un dispositif conforme à l'invention.

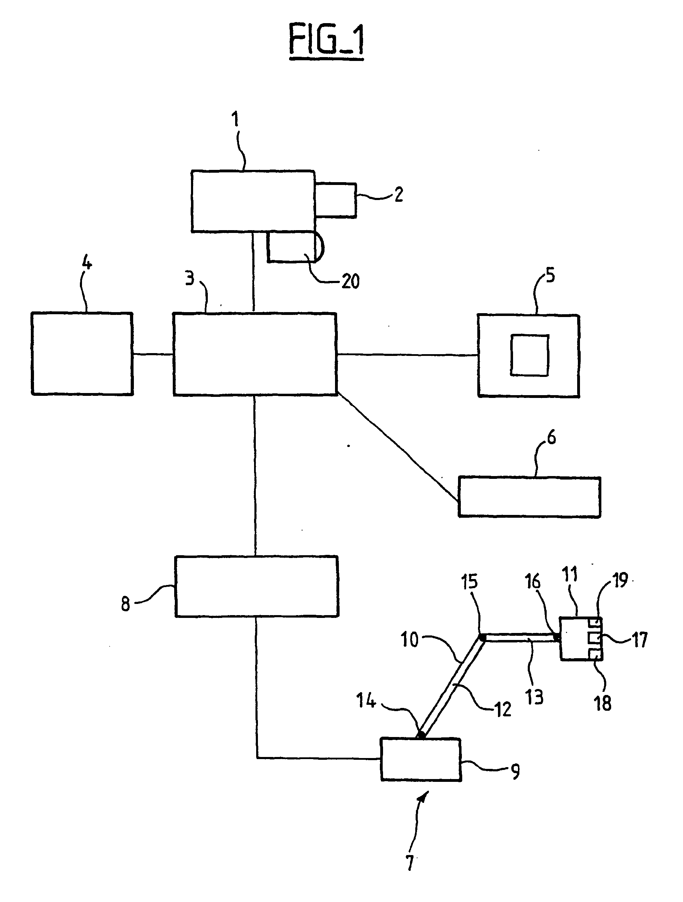

Comme on peut le voir sur la figure 1, le système de maquillage ou de coloration comprend au moins une caméra 1 équipée d'un objectif 2, une unité centrale 3 équipée d'une mémoire 4, d'un écran 5 et d'un clavier 6, et un applicateur 7 pourvu de moyens de commande 8. La caméra vidéo 1 peut être de type CCD. Alternativement, la caméra peut être de type classique, un moyen de conversion analogique/numérique étant alors prévu. La communication entre ces différents éléments peut être assurée par une liaison de type RS 232. La mémoire 4 et l'écran 5 peuvent être solidaires de l'unité centrale ou disposés dans des boîtiers séparés. La présence du clavier 6 est facultative et peut être remplacée par un écran 5 de type tactile permettant de réaliser des commandes. Une souris ou un dispositif du même genre peut aussi être prévue.

L'applicateur 7 comprend un boîtier 9 qui peut être fixé au sol ou sur tout support adapté, un ensemble articulé 10 fixé à une extrémité au boîtier 9 et supportant à l'extrémité opposée une tête d'application 11 de produit.

L'ensemble articulé 10 comprend deux bras 12 et 13. Le bras 12 est monté à pivotement sur le boîtier 9 par l'intermédiaire d'une articulation 14. Le bras 13 est monté à pivotement sur le bras 12 par une articulation 15 et la tête 11 est montée à pivotement sur le bras 13 par une articulation 16. Les articulations 14, 15 et 16 sont motorisées ou pourvues d'actionneurs permettant le déplacement de la tête 11 par rapport au boîtier 9 selon plusieurs axes, de préférence perpendiculaires les uns aux autres. De façon optionnelle, les bras 12 et 13 peuvent être télescopiques, par exemple au moyen d'un vérin électrique. Si l'on souhaite que la tête 11 effectue des mouvements plus complexes, on peut prévoir un nombre d'articulations supérieur à trois de façon qu'elles confèrent à ladite tête 11 un nombre de degrés de liberté plus élevé.

Une source de lumière 20 peut être fixée à la caméra 1 pour améliorer l'éclairage et donc la qualité des images obtenues. La source de lumière 20 sera active au moins en lumière visible et peut être de type diode électroluminescente, arc Xénon, halogène, etc.

La tête d'application 11 comprend une rangée de buses de projection 17 alimentées en produits de traitement à partir d'un ou plusieurs réservoir, non représenté, par exemple disposé dans le boîtier 9, et deux capteurs de distance 18 et 19 capables d'effectuer une mesure de la distance entre la tête d'application 11 et la surface sur laquelle le produit de traitement doit être appliqué. Les détecteurs 18 et 19 peuvent inclure chacun une diode laser émettant un faisceau laser réglé pour croiser l'autre faisceau laser émis par l'autre diode à la distance souhaitée entre la tête 11 et la surface devant recevoir le produit de traitement, de façon qu'un écart par rapport à cette distance souhaitée puisse être facilement détecté. Les buses 17 peuvent être du type à jet d'encre avec un cristal piézoélectrique ou un moyen électrostatique.

L'impression à jet d'encre est une méthode sans contact. L'encre est émise à partir de buses. Des encres liquides de différentes couleurs giclent sur la surface à traiter pour former une image. La tête d'application 11 balaie ladite surface par bandes parallèles. Pour accroître la vitesse d'impression, la tête d'application 11 imprime en une passe une rangée de pixels simultanément grâce à la rangée de buses 17. La technique à jet d'encre est généralement soit thermique, soit électrostatique, voire piézoélectrique.

Dans la présente application, on utilise de préférence la technique piézoélectrique dans laquelle un cristal piézoélectrique est disposé dans le fond d'un réservoir de produit à proximité d'une buse. Lorsqu'un courant est appliqué au cristal piézoélectrique, celui-ci se déforme, ce qui crée une force suffisante pour éjecter une gouttelette de produit. Le produit n'a pas à être chauffé et les gouttelettes peuvent être de taille très réduite. Pour l'obtention de dessins en couleur, on prévoira des cartouches de produit de couleurs cyan, magenta et jaune. De préférence, on prévoira également une cartouche de couleur noire afin d'obtenir un noir de bonne qualité. On pourrait aussi prévoir deux cartouches supplémentaires, cyan léger et magenta léger, pour des dessins plus fins.

Bien entendu, les caractéristiques du produit autre que celles de couleurs, seront adaptées à la partie du corps humain destinée à les recevoir : peau, ongles, cheveux, etc... Par ailleurs, des cartouches de produit de soin et/ou de produit de maquillage seront également prévues.

Le fonctionnement du système est le suivant. Une personne désirant traiter une partie de son corps, par exemple le visage, la main, la chevelure, etc..., est installée dans le champ de vision de la caméra 1 pour une ou plusieurs prises de vue. En effet, l'obtention d'une image tridimensionnelle de la partie du corps humain qui est une variante préférée, nécessite au moins deux prises de vue sous des angles différents au moyen d'au moins deux caméras fixes ou au moyen d'une caméra mobile. Les fichiers images obtenus lors de ces prises de vue sont transférés de la caméra 1 à l'unité centrale 3 qui effectue un traitement généralement appelé reconstruction et permettant d'obtenir une image tridimensionnelle qui est ensuite transférée à la mémoire 4. Pour améliorer la qualité de l'image tridimensionnelle, des traitements optionnels peuvent être effectués par l'unité centrale, par exemple pour corriger des défauts géométriques liés à la perception du relief. L'image tridimensionnelle obtenue et stockée dans la mémoire 4 peut être affichée à l'écran 5. Dans le cas d'une image bidimensionnelle, il n'est pas indispensable de prévoir un tel traitement par l'unité centrale 3.

Un logiciel de dessin stocké dans la mémoire 4 permet à l'utilisateur ou à un opérateur qui peut l'aider, de simuler sur l'écran 5 différents maquillages ou colorations possibles en fonction de dessins préexistants, stockés dans la mémoire 4, de dessins apportés par l'utilisateur sur un support numérique tel qu'une disquette ou un CD ROM aptes à être lus par un lecteur approprié, non représenté, relié à l'unité centrale 3. Des retouches peuvent être effectuées grâce au clavier 6 ou à l'écran 5 si celui-ci est du type tactile, jusqu'à l'obtention sur l'écran 5 de l'effet souhaité par l'utilisateur. Le dessin peut aussi être entièrement composé par l'utilisateur ou par l'opérateur. On peut utiliser des logiciels de dessin tels que PHOTOSHOP^{®} de la Société ADOBE ou PAINTBRUSH^{®} de la Société MICROSOFT.

En même temps que ce choix effectué par l'utilisateur, l'unité centrale effectue une analyse des caractéristiques de la surface à maquiller ou à colorer à partir de l'image bi ou tridimensionnelle, afin de déterminer les caractéristiques topologiques de ladite surface, pour déterminer si une application d'un produit de traitement est nécessaire préalablement à l'application d'un produit de maquillage ou de coloration, par exemple dans le cas d'une peau sèche ou encore dans le cas d'une peau ridée. L'image bidimensionnelle convient bien aux peaux sèches ou de couleurs hétérogènes (tache pigmentaire, cicatrice). Pour les peaux ridées, l'image tridimensionnelle est préférée.

L'unité centrale 3 effectue également un traitement permettant de reconnaître différentes parties du corps humain, notamment de différencier les cheveux de la peau, de reconnaître les doigts et les ongles d'une main et de reconnaître les différentes parties du visage, notamment les lèvres, les sourcils, les joues et plus généralement toute partie nécessitant l'application d'un produit de traitement, de maquillage ou de coloration spécifique, par exemple au moyen d'un logiciel de reconnaissance par segmentation.

A la suite de ces étapes, l'unité centrale 3 élabore un signal de commande de l'applicateur 7 et l'envoie aux moyens de commande 8. Les moyens de commande 8 en fonction du signal de commande reçu de l'unité centrale 3, commandent les opérations suivantes.

La tête d'application 11 est amenée dans le champ de vision de la caméra 1 et à proximité de la surface devant être maquillée ou colorée, de façon que la rangée de buses 17 se trouve à la distance souhaitée de projection, ce que l'on vérifie grâce aux capteurs 18 et 19. Bien entendu, la partie du corps de l'utilisateur devant être traitée sera convenablement immobilisée pendant la durée du traitement. Le contrôle dynamique de la distance tête-surface est effectué en temps réel par les moyens de commande 8.

Dans le cas d'une application de maquillage, un premier passage de la tête 11 peut permettre de déposer un produit de soin et un deuxième passage permettra de déposer le produit de maquillage proprement dit.

Avantageusement, on peut prévoir un troisième passage de la tête 11. Le deuxième passage permet alors de déposer un produit de couleur et le troisième passage de déposer un agent matifiant.

Dans le cas d'une tache pigmentaire détectée par la caméra 1 et repérée par l'unité centrale 3, le produit de traitement pourra être ou comprendre un produit recouvrant permettant de conférer à la tache pigmentaire le même aspect que le reste de la peau.

A titre de variante, on peut envisager de déposer simultanément deux ou un plus grand nombre de produits afin d'accélérer le processus. A cet effet, on peut prévoir plusieurs rangées de buses 17 disposées en matrice permettant d'appliquer simultanément mais en des lieux légèrement décalés des produits différents. En cas de présence de cicatrices, leur camouflage peut s'effectuer par application de colorant avec motifs en trompe-l'oeil, permettant de donner une illusion du relief. L'unité centrale 3 effectuera une analyse colorimétrique de l'image perçue par la caméra 1, de façon à déterminer la teinte locale de la partie devant être traitée.

On peut appliquer un produit de coloration, par exemple une encre, pour obtenir l'image sélectionnée par l'utilisateur sur l'écran 5. On peut ensuite prévoir une étape d'application d'un vernis et/ou d'un produit destiné à empêcher la desquamation et permettant de prolonger la tenue de la coloration. Bien entendu, on choisira une encre et éventuellement un vernis pouvant s'enlever sans dommage pour la peau, par exemple par un solvant organique, par de l'eau ou par un produit tensio-actif. On peut encore ajouter au produit de coloration ou de maquillage un auto-bronzant, par exemple de la dihydroxyacétone ou encore un ensemble substrat et enzyme réagissant in situ sur la peau lors de l'application, le substrat pouvant être un polymère de la famille des polyphénols.

Dans le cas de la coloration ou du maquillage d'un crâne dont les cheveux sont coupés extrêmement courts, on utilisera les produits destinés à la peau pour réaliser le motif souhaité par l'utilisateur, tel que zébrures, dégradés, rayures, etc...

Les buses de projection 17 peuvent être du type à projection piézoélectrique, permettant d'utiliser une grande variété de produits de traitement ou pigmentaires. Le produit est forcé à travers la buse qui est de faible diamètre et mis en vibration à haute fréquence par un cristal piézoélectrique disposé dans la tête 11. Le produit sous forme liquide se fractionne alors en fines gouttelettes qui sont expulsées par la buse. En sortie, les gouttelettes peuvent être déviées par tout moyen connu, tel qu'au moyen d'électrodes de déflection, ce qui permet une impression par jet continu multidévié.

L'ensemble de la surface à maquiller ou à colorer est parcouru par la tête 11 avec mesure temps réel de la distance grâce aux capteurs 18 et 19 de conservation de la distance nécessités par le type de buse que l'on utilise, ce qui permet un suivi précis du relief et une application de haute qualité.

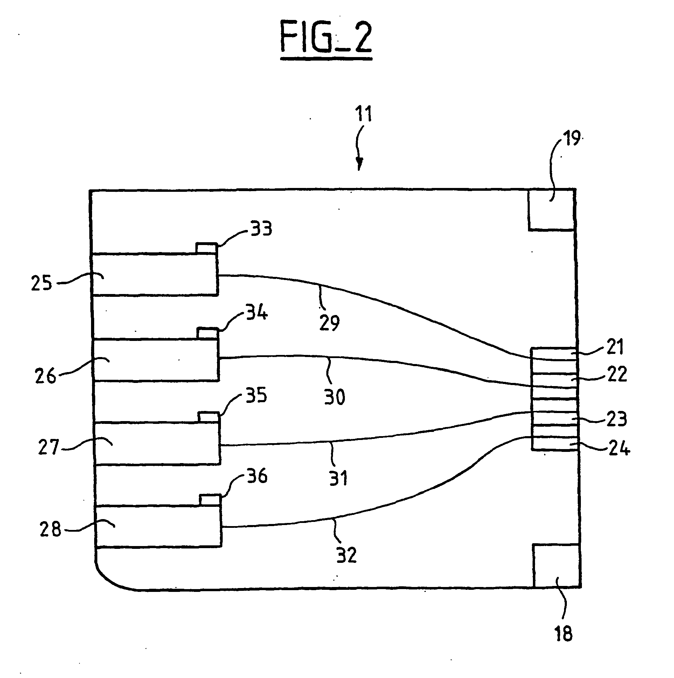

Sur la figure 2, est illustrée plus en détail la tête mobile 11. L'ensemble de buses 17 comprend quatre buses 21, 22, 23 et 24, quatre cartouches 25, 26, 27 et 28 amovibles, chacune contenant un produit que l'on souhaite appliquer et étant relié par une canalisation 29, 30, 31, 32 à la buse correspondante 21, 22, 23, 24. A titre d'exemple, les cartouches 25 à 28 peuvent contenir :
- chacune une composition colorante de base;
- chacune une composition colorante issue d'un mélange;
- l'une un produit couvrant du type fond de teint, les autres des compositions colorantes différentes, etc.

La tête mobile 11 comprend un moyen d'identification des cartouches 25 à 28, par exemple sous la forme de quatre capteurs 33 à 36, chacun dédié à une cartouche 25 à 28 et apte à reconnaître le contenu de la cartouche, notamment par lecture d'un code mécanique, optique, magnétique, etc.

Des moyens de pompage de produit sont également prévus pour transférer un produit d'une cartouche 25, 26, 27, 28 à la buse 21, 22, 23, 24 correspondante. La tête mobile 11 pourra comprendre un moyen de surveillance de la quantité de produit présente dans chaque cartouche 25, 26, 27, 28, par exemple sous la forme d'un capteur dédié à la mesure de la masse d'une cartouche 25, 26, 27, 28 et permettant une estimation du niveau de produit, ou d'un capteur dédié à la mesure du débit d'un moyen de pompage, ou encore d'un capteur dédié à la mesure du courant électrique consommé par un moyen de pompage, le courant diminuant lorsqu'une cartouche est vide et que le moyen de pompage ne voit plus passer de produit. Une cartouche vide pourra être signalée par l'affichage d'un message de cartouche vide sur l'écran 5 et/ou par l'émission d'un signal sonore. Alternativement, l'écran 5 pourra comprendre une zone dédiée à l'affichage du niveau de produit dans chaque cartouche.

La tête mobile 11 pourra comprendre un moyen de maintien en température si la nature des produits, notamment leur viscosité, l'exige, par exemple entre 20 et 27°C, mieux entre 22 et 24°C. Le maintien à une température relativement constante évite une dispersion de débit des buses du à une variation de viscosité.

Ainsi, après la sélection des caractéristiques visuelles souhaitées, le logiciel stocké dans la mémoire 4 et mis en oeuvre par l'unité centrale 3 vérifie que les cartouches présentes dans la tête 11 conviennent aux caractéristiques visuelles souhaitées. Si cela n'est pas le cas, un message d'alerte est affiché sur l'écran 5.

La vérification est effectuée d'après les signaux émis par les capteurs 33 à 35 et reçus par l'unité centrale 3, par exemple par liaison filaire passant par le boîtier 9.

Après que l'opérateur a disposé, le cas échéant, des cartouches convenables dans la tête 11, un message correspondant peut être affiché à l'écran 5.

Dans d'autres modes de réalisation, la tête 11 peut comprendre un moyen d'application tel qu'un aérographe ou un moyen d'application par décalcomanie, par tatouage ou par lingette imprégnée de produit à appliquer.

Le logiciel effectue le calcul des quantités instantanées de chaque produit pour chaque zone élémentaire de la partie à traiter en fonction des caractéristiques de ladite zone élémentaire. En d'autres termes, pour une zone élémentaire de coordonnées (x, y) ou (x, y, z), le logiciel calcule les quantités élémentaires partielles Q₂₅, Q₂₆, Q₂₇ et Q₂₈ de produits issus de cartouches 25, 26, 27 et 28 en fonction du type de chaque produit, des caractéristiques de la zone élémentaire et du résultat à obtenir qui peut être défini par des variables de couleur et de brillance.

Le logiciel détermine aussi l'ordre d'application des produits qui peut être successif sur une même zone ou juxtaposé sur des zones voisines. Dans certains cas, un seul produit sera appliqué et une seule cartouche sera utilisée même si d'autres restent en place sur la tête 11.

Afin d'avoir une corrélation directe entre les caractéristiques visibles de la peau et l'information mathématique bi ou tridimensionnelle, le logiciel pourra utiliser, par exemple, un procédé de mesure optique qui utilise une combinaison des techniques du code Gray et du décalage de phase. Il est possible avec cette méthode de déterminer avec une grande précision les coordonnées spatiales absolues de tous les points objets dans le domaine embrassé de l'image.

Dans la méthode du code Gray, les franges sont projetées successivement avec une modulation de luminosité rectangulaire et un nombre de lignes différent. Le nombre de lignes est doublé à chaque processus de projection, ce qui définit sans ambiguïté l'ordre des raies pour chaque point d'image. Dans la méthode du décalage des phases une seule frange est projetée plusieurs fois avec une modulation de luminosité sinusoïdale et une relation de phase différente. Ceci permet outre une reconstruction tridimensionnelle exacte de la surface pour laquelle chaque point d'image est défini indépendamment de ses voisins, un contrôle automatique de la qualité de mesure.

La résolution dans la direction verticale des Z typiquement avec 0,2% du domaine de mesure, conduit à une résolution effective de 4 µm en Z. Selon le type de caméra CCD employé, on atteindra une résolution de 45 µm dans la direction horizontale des X et Y. La séquence d'analyse d'image avec analyse des coordonnées correspondantes peut être réalisée en moins d'une seconde (typiquement 500-800 ms).

Les coordonnées tridimensionnelles de zone observée par la caméra 1 servent à positionner la tête 11 à une distance convenable (typiquement 1 cm) de la peau. Ceci est effectué par le contrôle d'une table de translation en Z au moyen de l'unité centrale 3.

L'image acquise par la caméra 1 pour le calcul des coordonnées tridimensionnelles de la zone observée, permet aussi de mesurer la couleur de la peau. Pour cela, la caméra 1 est calibrée colorimétriquement comme cela est fait pour un scanner en utilisant une image d'une mire de calibration et un logiciel de calibration, par exemple Profile Maker de la Société LOGO. Pour s'affranchir des phénomènes de brillance de la peau, on utilise des polariseurs en position croisée qui sont placés sur le projecteur 20 et devant l'objectif de la caméra. Cette procédure de calibration permet d'obtenir la correspondance de l'image acquise avec le système colorimétrique et indépendant de la caméra. L'image permet d'avoir la couleur sur chaque pixel donc en tout point de la zone observée.

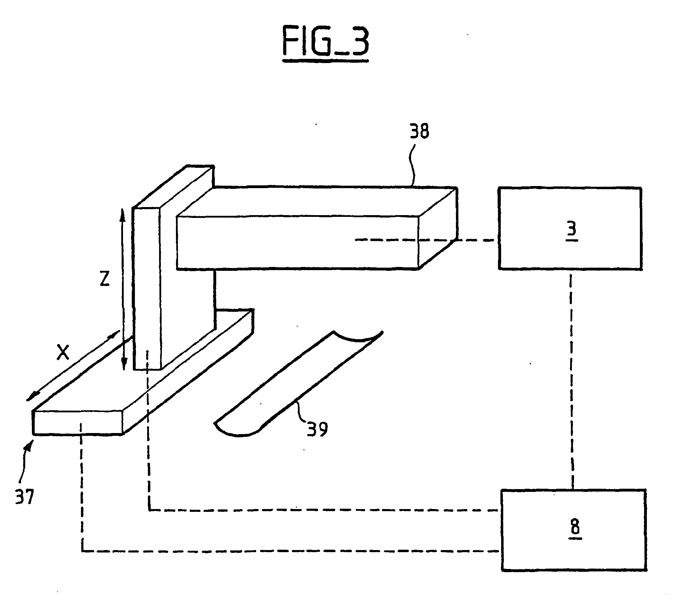

Le système de positionnement 37, illustré sur la figure 3, est composé de deux tables de translation avec moteurs pas à pas solidaires l'une de l'autre, pilotées via une unité de contrôle. Ces tables procurent le déplacement à la tête de distribution 38 en Z (distance à la zone d'application) et en X (translation le long de la zone d'application).

La distance de la tête de distribution 38 à la zone à traiter peut varier entre 20 µm et 10 cm, de préférence entre 100 µm et 5 cm, et de façon préférée, entre 250 µm et 1 cm.

La zone du corps à traiter est maintenue en place à l'aide d'un dispositif approprié. Citons par exemple pour le bras d'une gouttière 39 et pour la tête d'une mentonnière, non représentée.

Un logiciel de dessin supportant les calibrations colorimétriques d'écran et d'impression (type Photoshop V5.02 de la Société ADOBE), permet de sélectionner à partir d'une base de données d'images le type de maquillage à appliquer sur la zone et de visualiser le rendu après application. L'image acquise par la caméra calibrée permet l'exacte restitution des couleurs de la zone.

Grâce au logiciel de modification d'image, il est possible de corriger sélectivement un défaut de la zone à traiter (exemple : zone dépigmentée). Cette zone est détourée à l'aide de la souris et peut être corrigée en utilisant la même couleur que la peau environnante.

Les couleurs sont obtenues via le mélange des quatre couleurs : cyan, magenta, jaune, noir. La tête est calibrée colorimétriquement (profil ICC) de façon à restituer parfaitement les couleurs du maquillage sélectionné et visualisé à l'écran.

Dans un mode préféré de l'invention, un contrôle dynamique de la position de la tête de distribution 38 par rapport à la peau peut être effectué grâce à un système de télémesure. Par exemple, en utilisant un capteur de distance sans contact opérant sur le principe de la triangulation. Le capteur a une dynamique de mesure de plus ou moins 1 cm avec une précision inférieure à 10 µm et une distance de travail de 6,5 cm, par exemple de type BULLIER INTERNATIONAL, Référence M5L/20. Le signal du capteur est numérique et est relié à l'unité centrale. L'asservissement de la position de la tête de distribution permet de compenser des faibles mouvements de la zone à traiter. L'asservissement est effectué par l'unité centrale pendant l'impression du dessin sélectionné.

Grâce à l'invention, on peut réaliser un maquillage, une coloration ou un soin adaptés aux desideratas de l'utilisateur. On entend ici par "maquillage" l'application de produit tenant compte des caractéristiques colorimétriques et topologiques de la peau, et par "coloration" l'application de produit couvrant camouflant totalement la teinte d'origine de la peau. Le système s'applique également à la teinture des cheveux. On peut y réaliser des motifs de différentes couleurs et de différentes formes au moyen de cette machine de traitement automatique.

La présence de plusieurs cartouches permet d'éviter la formation d'un mélange de produits préalablement à l'application. Au contraire, chaque produit est appliqué directement. On évite ainsi l'utilisation d'une quantité excessive de produits dont le mélange est spécifique à une personne ou à un endroit localisé d'une personne et ne peut être utilisé ailleurs.

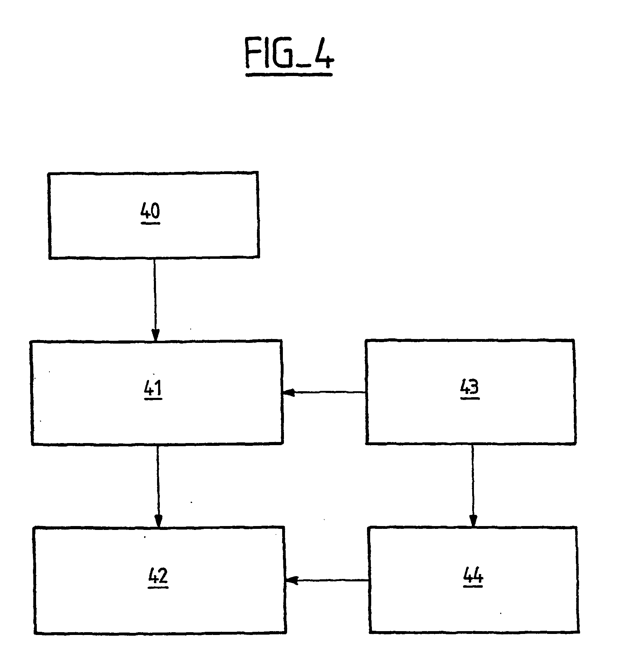

La figure 4 illustre les différentes étapes du procédé d'acquisition d'images, notamment dans le cas de deux caméras identiques regardant un objet par deux voies optiques différentes. Les deux images appelées image gauche et image droite sont acquises en même temps, car les deux caméras sont synchronisées. La prise de vue est immédiate, ce qui supprime les éventuels problèmes de mouvement de l'utilisateur. Le volume de travail est limité par la taille des champs verticaux et horizontaux des caméras ainsi que par la profondeur de champ des objectifs. Les deux vues présentent des disparités que l'on peut quantifier, qui permettent de remonter à la topographie de la surface observée. Le calcul de la topographie de l'objet observé se fait à l'étape 40 par acquisition des images gauche et droite, à l'étape 41 par le calcul de la géométrie optique avec interpolation et redressement des images assurés par l'unité centrale 3 de la figure 1, à l'étape 42 de calcul des disparités entre les images gauche et droite et par calcul de la topographie de la surface.

A titre d'exemple, à l'étape 43, les données d'étalonnage sont obtenues à l'aide d'une mire que l'on déplace dans le volume de travail des deux caméras, l'étalonnage étant fait sur une centaine de points par plan sur plusieurs plans séparés d'un pas, par exemple de quelques mm en fonction de l'objet à mesurer. On en extrait des paramètres extrinsèques des caméras relatifs aux positions et orientations par rapport au repère d'étalonnage, et des paramètres intrinsèques des caméras (caractéristiques optiques) liés au modèle de sténopé et de distorsion associés. Cet étalonnage est fait une fois pour toutes et définit la géométrie des deux caméras. Le calcul des disparités entre les caméras est réalisé à deux échelles différentes. Puis on recherche le minimum de corrélation entre les deux vues aux deux échelles décrites. La position de ce minimum est interpolée paraboliquement, ce qui donne une précision suffisante dont l'erreur est inférieure au pixel. L'altitude du point retenu est inversement proportionnelle à la position du minimum calculée. Les données d'étalonnage calculées à l'étape 43 sont fournies lors de l'étape 41 de calcul de la géométrie optique et lors d'une étape 44 de calcul de nouveaux paramètres des caméras, lesquelles sont fournies à l'unité centrale lors de l'étape 42 de calcul de topographie de la surface.

Le produit de traitement susceptible d'être appliqué dans le cadre de la présente invention, peut être de toute nature cosmétiquement acceptable.

Il peut s'agir d'un produit de maquillage, de coloration, susceptible d'être appliqué sur la peau du corps et/ou du visage, sur les cheveux, les cils, les sourcils, les poils et/ou les ongles.

Il peut ainsi comprendre les ingrédients usuels en cosmétique, tels que par exemple des huiles, des cires, de l'eau, des solvants, des matières colorantes, des épaississants, des tensio-actifs, des charges, des polymères filmogènes ou non.

Parmi les huiles et/ou les cires, on peut citer les huiles et cires hydrocarbonées et/ou siliconées et/ou fluorées, d'origine animale, végétale, minérale ou synthétique, volatiles ou non.

Parmi les solvants, on peut citer les alcools, notamment en C₁-C₆ tels que l'éthanol ou l'isopropanol; les glycols tels que l'éthylène glycol ou le propylène glycol; le glycérol; les éthers de propylène glycol; les cétones; les esters; les éthers; les alcanes; les composés cycliques aromatiques (toluène, benzène, xylène); les aldéhydes.

Dans un mode de réalisation préféré, il peut comprendre au moins une matière colorante qui peut être un pigment ou un colorant hydrosoluble ou liposoluble.

Les pigments peuvent être blancs ou colorés, minéraux et/ou organiques, de taille micrométrique ou nanométrique.

On peut citer, parmi les pigments et nanopigments minéraux, les oxydes de titane, de zirconium ou de cérium, ainsi que les oxydes de zinc, de fer ou de chrome, le bleu ferrique, l'hydrate de chrome, les outremers (polysulfures d'aluminosilicates).

Parmi les pigments organiques, on peut citer le noir de carbone, et les laques telles que les sels de calcium, de baryum, d'aluminium, de zirconium ou de strontium.

Parmi les colorants hydrosolubles, on peut citer les colorants usuels du domaine considéré tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, et leurs mélanges. On peut encore citer les colorants halogéno-acides, azoïques, anthraquinoniques.

Le produit peut par ailleurs comprendre les additifs usuels couramment employés dans le domaine considéré, tels des antioxydants, des parfums, des conservateurs, des actifs cosmétiques, des vitamines, des acides gras essentiels, des sphingolipides, des composés autobronzants tels que la DHA, des filtres solaires.

Dans un mode de réalisation préféré, le produit comprend au moins un composé généralement utilisé pour la coloration temporaire ou permanente, des cheveux ou des ongles.

Dans un autre mode de réalisation préféré, le produit peut comprendre au moins un composé susceptible de colorer de manière temporaire la peau, tel que des auto-bronzants (DHA notamment).

Dans un autre mode de réalisation préféré, le produit peut comprendre au moins un actif cosmétique, notamment un actif de soin tel qu'un hydratant, un agent de blanchiment de la peau, un produit de soin pour tache pigmentaire, un agent prodésquamant, un antirides.

Le produit est destiné à être appliqué sur la peau du visage et du corps, sur les muqueuses et/ou sur les fibres kératiniques, telles que les ongles, les cils ou les cheveux. Il peut se présenter sous toutes les formes galéniques envisageables, telles que gel huileux ou aqueux; émulsion liquide ou gélifiée, huile-dans-eau, eau-dans-huile ou multiple; dispersion; système multiphases, notamment biphase; composition solide telle que stick ou bâton.

Ce produit peut se présenter comme composition d'hygiène corporelle; comme composition capillaire, par exemple comme stick de coiffage ou stick de maquillage des cheveux; comme composition de maquillage de la peau du visage, du corps ou des lèvres, par exemple comme rouge à lèvres, fond de teint coulé en stick ou en coupelle, fard à joues ou paupières, base fixante à appliquer sur un rouge à lèvres classique, stick anti-cernes, brillant à lèvres, eye-liner, mascara, produits de tatouage éphémère; comme composition de soin de la peau du visage, du corps y compris le cuir chevelu, des lèvres, des cheveux ou des ongles, par exemple comme baume ou base de soin pour les lèvres, crème de soin journalier, composition matifiante; comme composition solaire ou auto-bronzante.

Dans un mode de réalisation de l'invention, la caméra 1, l'applicateur 7 et un écran sont disposés dans un premier lieu, et une unité de traitement de données équipée de ses périphériques est disposée dans un deuxième lieu et est reliée à la caméra 1 et l'applicateur 7 par un réseau de communication, par exemple de type internet. Des interfaces tels que des modems seront reliés à la caméra 1 et l'applicateur 7 d'une part et à l'unité de traitement de données d'autre part. L'unité de traitement de données peut être associée à une base de donnée ce qui permet un choix de dessin plus large. Par ailleurs, l'unité de traitement de données peut être à puissance de calcul élevée. L'unité de traitement de données envoie une ou plusieurs images vers le premier lieu dans lequel l'utilisateur effectue un choix d'image et marque son accord. L'élaboration du signal de commande peut alors être effectué dans le premier lieu par un moyen local de traitement de données, ou dans le second lieu par ladite unité de traitement de données à réception d'un signal représentatif du choix de l'utilisateur.

En variante, l'utilisateur peut effectuer son choix d'image en un troisième lieu, par exemple à son domicile, sur un ordinateur personnel connecté à un réseau de communication, marque son accord sur une image, accord qui est transmis à ladite unité de traitement de données qui élabore alors un signal destiné à commander l'applicateur, et l'envoie. L'applicateur, à réception dudit signal, est prêt à réaliser l'image. L'utilisateur se déplace alors du troisième lieu vers le deuxième lieu, par exemple un magasin de cosmétique, manucure, une pharmacie, etc.

Alternativement, l'utilisateur dispose de l'applicateur à son domicile. Il peut aussi disposer de la caméra à domicile. Dans ce cas, l'image prise par la caméra est envoyée à une unité distante de traitement de données qui effectue les différentes opérations nécessaires, propose au moins une image, élabore et envoie un signal destiné à commander l'applicateur à réception de l'accord de l'utilisateur.

Dans un mode de réalisation de l'invention, la caméra 1 et l'applicateur 7 sont disposés, avec un ordinateur pourvu d'un écran et d'un clavier 6, dans une zone d'acquisition et d'application, située par exemple chez l'utilisateur ou dans une boutique d'esthéticien. Un ordinateur et une base de données sont disposés dans une zone distante, située par exemple chez le concepteur ou chez un exploitant du système. Cet ordinateur et la base de données sont reliés par une liaison informatique, de préférence à débit de données élevé, tout en pouvant être situés à distance l'un de l'autre. Les deux ordinateurs sont reliés par un réseau de télécommunication du type internet, de préférence un internet rapide par exemple ADSL. L'ordinateur associé à la base de données pourra avoir une forte puissance de calcul et en faire bénéficier l'utilisateur, tandis que l'ordinateur associé à la caméra sera de type ordinateur grand public bon marché.

En outre, est prévu une unité de formulation de produits, par exemple dans un laboratoire, reliée à la base de données par une liaison informatique, de préférence à débit de données élevé, tout en pouvant être situées à distance l'une de l'autre.

Dans un sens, des données acquises par la caméra 1 dans la zone d'acquisition et d'application seront envoyées à l'ordinateur associé à la base de données par l'intermédiaire de l'ordinateur associé à la caméra qui peut ou non effectuer un premier traitement des données. Les données peuvent servir à alimenter une base de données regroupant toutes les typologies de clientes actuelles ou potentielles. Les données contenues dans cette base peuvent ensuite être utilisées par un formulateur de produits cosmétiques afin de déterminer les caractéristiques d'un produit qu'il conviendrait de développer pour répondre aux besoins de cette clientèle.

Dans l'autre sens, est véhiculé un signal contenant des informations destinées à l'applicateur 7. Plus particulièrement, l'ordinateur associé à la base de données effectue un traitement de données pour générer un message d'application et/ou des données d'une ou plusieurs images calculées d'après des caractéristiques de l'utilisateur. Si l'ordinateur associé à la caméra reçoit le message d'application, il le traite, par exemple par décompression si ledit message contient des données comprimées, et envoie un signal de commande à l'applicateur 7. Ledit envoi du signal de commande peut être soumis à une validation de l'utilisateur.

Si l'ordinateur associé à la caméra reçoit des données d'une image, il l'affiche sur l'écran 5. L'ordinateur peut ensuite requérir une validation de l'utilisateur. Après ladite validation, l'ordinateur peut envoyer à l'ordinateur associé à la base de données une requête de message d'application.

Dans un autre mode de réalisation, la caméra 1, et son ordinateur associé, sont disposés dans une zone d'acquisition située par exemple chez l'utilisateur. L'applicateur 7 est disposé dans une zone d'application, située par exemple dans une boutique d'esthéticien. L'applicateur 7 est relié à l'ordinateur associé à la base de données par une liaison du même type que celle prévue entre l'ordinateur associé à la caméra et l'ordinateur associé à la base de données.

En d'autres termes, les zones d'acquisition et d'application sont distinctes et peuvent être distantes. Il peut être prévu un code personnel pour identifier des caractéristiques propres à l'utilisateur ou des caractéristiques choisies par l'utilisateur et stockées dans la base de données. Le code personnel peut être indiqué à l'utilisateur par l'ordinateur associé à la caméra dans la zone d'acquisition et lui sera demandé par l'ordinateur associé à la base de données dans la zone d'application. Le code personnel peut posséder une double fonction d'identification de données d'un utilisateur et de protection de la confidentialité desdites données.

## Revendications

1. Procédé automatisé de maquillage, coloration ou réalisation de dessins sur une partie du corps humain par projection d'un ou plusieurs produits colorés, comportant une étape d'acquisition d'au moins une image de ladite partie du corps humain avec une caméra, **caractérisé par le fait qu'**il comprend les étapes supplémentaires
- d'analyse topologique et colorimétrique et/ou de l'état de surface de ladite partie du corps humain à partir d'éléments unitaires de l'image de détermination des coordonnées en 3 dimensions de la dite partie du corps humain observée par la caméra
- et d'application du maquillage, de la coloration ou du dessin sur ladite partie du corps humain en asservissant la position d'une tête mobile comprenant au moins un capteur de distance et au moins une buse de projection de produit coloré, ladite position étant maintenue à une distance de ladite partie du corps humain entre 20 microns et 5 cm sur la base d'une mesure de distance entre la tête mobile et ladite partie du corps humain effectuée par ledit capteur de distance,
- avec éventuellement correction colorimétrique de certaines zones en utilisant la même couleur que la zone environnante.

2. Procédé selon la revendication 1, comprenant une étape de reconnaissance des caractéristiques visuelles souhaitées, l'application du maquillage, de la coloration ou du dessin étant faite en fonction desdites caractéristiques souhaitées.

3. Procédé selon la revendication 1 ou 2, comprenant des étapes de définition de zones particulières de ladite partie du corps humain et l'application de produits successivement sur lesdites zones particulières.

4. Procédé selon l'une quelconque des revendications précédentes, comprenant une étape de mémorisation pour une personne donnée des produits utilisés et des caractéristiques visuelles obtenues.

5. Procédé selon l'une quelconque des revendications précédentes, dans lequel on surveille les caractéristiques locales obtenues immédiatement après l'application des produits.

6. Procédé selon la revendication 5, dans lequel la surveillance des caractéristiques locales obtenues immédiatement après l'application des produits est effectuée à partir de la tête mobile.

7. Procédé selon l'une quelconque des revendications précédentes, dans lequel on reconstruit tridimensionnellement ladite partie du corps humain pour obtenir une représentation tridimensionnelle.

8. Procédé selon l'une quelconque des revendications précédentes, dans lequel on effectue les étapes de prise d'image et d'application dans une première zone, et l'étape d'analyse dans une deuxième zone distincte de la première zone, avec une communication entre lesdites zones par un réseau de télécommunications, du type internet.

9. Procédé selon l'une quelconque des revendications 1 à 7, dans lequel on effectue l'étape de prise d'image dans une première zone, l'étape d'analyse, dans une deuxième zone distincte de la première zone, et l'étape d'application dans une troisième zone distincte des première et deuxième zones, avec une communication entre lesdites zones par un réseau de télécommunications, du type internet.

10. Dispositif de maquillage, coloration ou réalisation de dessins sur une partie du corps humain par projection d'un ou plusieurs produits colorés, comprenant une caméra de prise de vue et d'acquisition d'au moins une image de ladite partie du corps humain, **caractérisé par le fait qu'**il comprend :
- un moyen d'analyse topologique et colorimétrique et/ou de l'état de surface de ladite partie du corps humain à partir d'éléments unitaires de l'image
- un moyen de détermination des coordonnées en 3 dimensions de la dite partie du corps humain observée par la caméra
- et un moyen d'application du maquillage, de la coloration ou du dessin sur ladite partie du corps humain comprenant une tête mobile comprenant au moins un capteur de distance et au moins une buse de projection, ledit moyen d'application comportant des moyens pour asservir la position de la tête mobile de façon que la distance de la tête mobile par rapport à ladite partie du corps humain soit maintenue comprise entre 20 microns et 5 cm sur la base d'une mesure de distance entre la tête mobile et ladite partie du corps humain effectuée par ledit capteur de distance.

11. Dispositif selon la revendication 10, **caractérisé par le fait que** le moyen d'application est supporté par un bras articulé pour être apte à suivre le relief de ladite partie.

12. Dispositif selon la revendication 10 ou 11, **caractérisé par le fait que** ladite tête est de type piézoélectrique, pneumatique, électrospray, thermospray ou aérosol.

13. Dispositif selon l'une quelconque des revendications 10 à 12, **caractérisé par le fait qu'**il comprend des moyens d'asservissement de la quantité totale et des quantités partielles de chaque produit en fonction des caractéristiques visuelles souhaitées.

14. Dispositif selon l'une des revendication 10 à 13 comprenant un moyen de correction colorimétriques sur certaines zones.

15. Programme d'ordinateur comprenant des moyens de code programme pour mettre en oeuvre les étapes du procédé selon l'une quelconque des revendications 1 à 9, lorsque ledit programme fonctionne sur un ordinateur.

## Claims

1. Automated process for applying makeup, for dyeing or for producing designs over a human body part by spraying one or more dyed products, comprising an image acquisition step of acquiring at least one image of said human body part with a camera, **characterized in that** it comprises the following additional steps:
- topological and colorimetric analysis, and/or analysis of the state of the surface of said human body part, from unit elements of the image;
- determination of coordinates in three dimensions of said human body part observed by the camera; and
- application of the makeup, dye or design over said human body part by controlling the position of a moving head comprising at least one distance sensor and at least one spraying nozzle for spraying the dyed product, said position being kept at a distance of between 20 microns and 5 cm from said human body part, based on a measurement of distance between the moving head and said human body part carried out by said distance sensor; and
- with optional colorimetric correction of certain areas using the same colour as the surrounding area.

2. Process according to Claim 1, comprising a step of recognizing desired visual characteristics, the application of the makeup, the dye or the design being carried out according to said desired characteristics.

3. Process according to Claim 1 or 2, comprising steps of defining particular areas of said human body part and applying products successively over said particular areas.

4. Process according to any one of the preceding claims, comprising a step of storing, for a given person, the products used and the visual characteristics obtained.

5. Process according to any one of the preceding claims, in which the local characteristics obtained immediately after the application of the products are monitored.

6. Process according to Claim 5, in which the monitoring of the local characteristics obtained immediately after the application of the products is carried out using the moving head.

7. Process according to any one of the preceding claims, in which said human body part is reconstructed three-dimensionally in order to obtain a three-dimensional representation.

8. Process according to any one of the preceding claims, in which the image taking and application steps are carried out in a first zone, and the analysis step is carried out in a second zone distinct from the first zone, with communication between said zones by means of a telecommunications network, of the Internet type.

9. Process according to any one of Claims 1 to 7, in which the image taking step is carried out in a first zone, the analysis step is carried out in a second zone distinct from the first zone, and the application step is carried out in a third zone distinct from the first and second zones, with communication between said zones by means of a telecommunications network, of the Internet type.

10. Device for applying makeup, for dyeing or for producing designs over a human body part by spraying one or more dyed products, comprising a camera for view-taking and acquisition of at least one image of said human body part, **characterized in that** it comprises:
- a means of topological and colorimetric analysis, and/or of analysis of the state of the surface of said human body part, from unit elements of the image;
- a means for determination of coordinates in three dimensions of said human body part observed by the camera; and
- an application means for applying the makeup, dye or design over said human body part comprising a moving head comprising at least one distance sensor and at least one spraying nozzle, said application means comprising means for controlling the position of the moving head so that the distance of the moving head relative to said human body part is kept at between 20 microns and 5 cm, based on a measurement of distance between the moving head and said human body part carried out by said distance sensor.

11. Device according to Claim 10, **characterized in that** the application means is supported by an arm which is articulated in order to be capable of following the relief of said part.

12. Device according to Claim 10 or 11, **characterized in that** said head is of the piezoelectric, pneumatic, electrospray, thermospray or aerosol type.

13. Device according to any one of Claims 10 to 12, **characterized in that** it comprises means for automatically controlling the total amount and the partial amounts of each product according to the visual characteristics desired.

14. Device according to any one of Claims 10 to 13, comprising a means for colorimetric correction over certain areas.

15. Computer program comprising program code means to implement the steps of the process according to any one of Claims 1 to 9, when said program runs on a computer.

## Patentansprüche

1. Automatisiertes Verfahren zum Schminken, Färben oder zur Erzeugen von Bildern auf einem Teil des menschlichen Körpers durch Aufspritzen eines oder mehrerer farbiger Produkte, das einen Schritt umfasst, in dem mindestens ein Bild dieses Teils des menschlichen Körpers mit einer Kamera erfasst wird, **dadurch gekennzeichnet, dass** es die folgenden zusätzlichen Schritte umfasst:
- topologische und kolorimetrische Analyse und/oder Analyse des Zustands der Oberfläche dieses Teils des menschlichen Körpers ausgehend von Einheitselementen des Bildes,
- Bestimmung der Koordinaten in 3 Dimensionen dieses Teils des menschlichen Körpers, der durch die Kamera beobachtet wird,
- und Auftragen der Schminke, der Färbung oder des Bildes auf diesen Teil des menschlichen Körpers, indem die Position eines beweglichen Kopfes, der mindestens einen Abstandssensor und mindestens eine Düse für das Aufspritzen des farbigen Produkts umfasst geregelt wird, wobei die Position auf der Basis einer Messung des Abstands zwischen dem beweglichen Kopf und diesem Teil des menschlichen Körpers, die mit dem Abstandssensor durchgeführt wird, in einem Abstand zu diesem Teil des menschlichen Körpers im Bereich von 20 µm bis 5 cm gehalten wird,
- gegebenenfalls mit einer kolorimetrischen Korrektur bestimmter Zonen unter Verwendung der gleichen Farbe wie in der umgebenden Zone.

2. Verfahren nach Anspruch 1, das einen Schritt umfasst, in dem gewünschte visuelle Merkmale identifiziert werden, wobei die Schminke, die Färbung oder das Bild in Abhängigkeit von diesen gewünschten Merkmalen aufgetragen wird.

3. Verfahren nach Anspruch 1 oder 2, das Schritte umfasst, in denen besondere Zonen dieses Teils des menschlichen Körpers definiert werden und nacheinander Produkte auf die besonderen Zonen aufgetragen werden.

4. Verfahren nach einem der vorhergehenden Ansprüche, das einen Schritt umfasst, in dem für eine gegebene Person die verwendeten Produkte und die erhaltenen visuellen Merkmale gespeichert werden.

5. Verfahren nach einem der vorhergehenden Ansprüche, in dem die erhaltenen lokalen Merkmale unmittelbar nach dem Auftragen der Produkte überwacht werden.

6. Verfahren nach Anspruch 5, in dem die Überwachung der unmittelbar nach dem Auftragen der Produkte erhaltenen lokalen Merkmale mit dem beweglichen Kopf durchgeführt wird.

7. Verfahren nach einem der vorhergehenden Ansprüche, in dem der Teil des menschlichen Körpers dreidimensional rekonstruiert wird, um eine dreidimensionale Darstellung zu erhalten.

8. Verfahren nach einem der vorhergehenden Ansprüche, in dem die Schritte der Bildaufnahme und des Auftragens in einer ersten Zone und der Analyseschritt in einer zweiten Zone, die von der ersten Zone verschieden ist, unter Kommunikation zwischen diesen Zonen mit Hilfe eines Telekommunikationsnetzwerks vom Typ Internet durchgeführt werden.

9. Verfahren nach einem der Ansprüche 1 bis 7, in dem der Schritt der Bildaufnahme in einer ersten Zone, der Analyseschritt in einer zweiten Zone, die von der ersten Zone verschieden ist, und der Schritt des Auftragens in einer dritten Zone, die von der ersten Zone und der zweiten Zone verschieden ist, unter Kommunikation zwischen diesen Zonen mit Hilfe eines Telekommunikationsnetzwerks vom Typ Internet durchgeführt werden.

10. Vorrichtung zum Schminken, Färben oder zur Erzeugung von Bildern auf einem Teil des menschlichen Körpers durch Aufspritzen eines oder mehrerer farbiger Produkte, die eine Kamera zur Aufnahme und zur Erfassung mindestens eines Bildes dieses Teils des menschlichen Körpers umfasst, **dadurch gekennzeichnet, dass** die Vorrichtung umfasst:
- eine Einrichtung zur topologischen und kolorimetrischen Analyse und/oder zur Analyse des Zustands der Oberfläche dieses Teils des menschlichen Körpers ausgehend von Einheitselementen des Bildes,
- eine Einrichtung zur Bestimmung der Koordinaten in 3 Dimensionen dieses Teils des menschlichen Körpers, der durch die Kamera beobachtet wird,
- und eine Einrichtung zum Auftragen der Schminke, der Färbung oder des Bildes auf diesen Teil des menschlichen Körpers, die einen beweglichen Kopf umfasst, der mindestens einen Abstandssensor und mindestens eine Spritzdüse umfasst, wobei die Einrichtung zum Auftragen Einrichtungen zur Regelung der Position des beweglichen Kopfes umfasst, durch die, auf der Basis einer Messung des Abstands zwischen dem beweglichen Kopf und dem Teil des menschlichen Körpers, die mit dem Abstandssensor durchgeführt wird, der Abstand des beweglichen Kopfes, bezogen auf diesen Teil des menschlichen Körpers, im Bereich von 20 µm bis 5 cm gehalten wird.

11. Vorrichtung nach Anspruch 10, **dadurch gekennzeichnet, dass** die Einrichtung zum Auftragen von einem beweglichen Arm getragen wird, damit sie imstande ist, dem Relief dieses Teils zu folgen.

12. Vorrichtung nach Anspruch 10 oder 11, **dadurch gekennzeichnet, dass** der Kopf ein Kopf vom piezoelektrischen Typ, pneumatischen Typ, Elektrosprühtyp, Thermosprühtyp oder Aerosoltyp ist.

13. Vorrichtung nach einem der Ansprüche 10 bis 12, **dadurch gekennzeichnet, dass** sie Einrichtungen zur Regelung der Gesamtmenge und der Teilmengen aller Produkte in Abhängigkeit von den gewünschten visuellen Merkmalen umfasst.

14. Vorrichtung nach einem der Ansprüche 10 bis 13, die eine Einrichtung zur kolorimetrischen Korrektur auf bestimmten Zonen umfasst.

15. Computerprogramm, das einen Programmcode umfasst, mit dem die Schritte des Verfahrens nach einem der Ansprüche 1 bis 9 durchgeführt werden, wenn das Programm in einem Computer in Betrieb ist.
